# EUROPEAN PATENT APPLICATION

(11) **EP 2 985 276 A1**
(43) Date of publication of application: **17.02.2016**
(21) Application number: 14754920.8
(22) Date of filing: 20.02.2014
(51) Int. Cl.: C07C 233/18, A61K 31/16, B82B 1/00, B82Y 5/00

(54) **FORMULATIONS BASED ON NANOEMULSIONS AND THE USE THEREOF FOR TREATING OBESITY**

(30) Priority: 20.02.2013 ES 201330233
(71) Applicant: Fundación Pública Andaluza Para La Investigación de Malaga en Biomedicina y Salud (FIMABIS), 29010 Málaga (ES); Universidad de Granada, 18071 Granada (ES)
(72) Inventor: RODRIGUEZ DE FONSECA, Fernando, E-29010 Málaga (ES); PAVÓN MORÓN, Francisco Javier, E-29010 Málaga (ES); SERRANO CRIADO, Antonia, E-29010 Málaga (ES); ROMERO CUEVAS, Miguel, E-29010 Málaga (ES); WULFF PÉREZ, Miguel, E-18071 Granada (ES); GÁLVEZ RUIZ, María José, E-18071 Granada (ES); MARTÍN RODRÍGUEZ, Antonio, E-18071 Granada (ES); DE VICENTE ÁLVAREZ-MANZANEDA, Juan, E-18071 Granada (ES)
(74) Representative: Hoffmann Eitle
(86) International application number: PCT/ES2014/070129
(87) International publication number: WO 2014/128333

(57) **Abstract**

The present invention is of interest for the pharmaceutical field. It refers to novel therapeutic formulations based on nanoemulsions of vegetal oils as delivery systems for compounds derived from N-acylethanolamines (NAEs) for their administration as pharmacological tool and as medication to induce satiety and food intake control, to regulate pro-anorexigenic and metabolic effects, and to prevent weight gain.

## Description

The present invention is of interest for the pharmaceutical field. It relates to novel therapeutic formulations based on nanoemulsions of vegetal oils as transport systems for compounds derived from N-acylethanolamines (NAEs) for their administration as pharmacological tool and as medication to induce satiety and food intake control, to regulate pro-anorexigenic and metabolic effects, and to prevent weight gain.

### PRIOR ART

In the past 20 years, the incidence of obesity has grown to reach an epidemic proportion during 21st century in practically all countries according to World Health Organization (WHO). In 2011, this organization indicated that overweight and obesity were the fifth main risk factor for death in the world with 2.8 millions of deaths of adult people (WHO, 10 facts on obesity, 2010) and an estimation of 2,300 millions of adults with overweight and 700 millions with obesity for 2015. The impact on the public health system is enormous because obesity is related to cardiovascular, metabolic (diabetes, cholesterol, triglycerides...), dermatological, gastrointestinal and osteoarticular diseases and/or certain types of cancer such as colon cancer. According to WHO, a 44% of type 2 diabetes is attributable to obesity or overweight (WHO. Fact sheet N° 311, 2011).

One of the strategies used to solve this problem is the use of pharmacological tools with different mechanisms of action and aimed at different biological targets. However, this line of treatment is not fully consolidated due to the problems of side effects, which have produced the withdrawal of many drugs, previously authorized by the main agencies for the safety of drugs (USFDA, USA; and EMA, Europe), from the market. For example:
a) *Rimonabant* (SR141716A, Acomplia® and Zimulti® from Sanofi-Aventis) is the first cannabinoid CB1 receptor antagonist/inverse agonist (related to NAEs) approved in 2006 for the treatment of both obese and overweight patients with associated risk factors, such as type 2 diabetes or (hyper)lipidemia. However, psychiatric side effects such as depressive and anxiety disorders (as well as a higher risk of suicide) were observed in clinical studies. For this reason it was withdrawn from market IN 2007 (USA) and 2008 (Europe).
b) A dual/central reuptake inhibitor of serotonin and norepinephrine, named *sibutramine* (Meridia® or Reductil® from Abbott Laboratories), with cardiovascular effects. In fact, it was demonstrated that produces myocardial infarction and ictus in overweight patients with a preexisting cardiovascular disease. Consequently it was withdrawn in 2010.

Therefore, the current pharmacological tools against obesity continue being reduced:
a) A lipase inhibitor named *orlistat* (Xenical® from Roche) with gastrointestinal side effects: flatulence, diarrhea, abdominal pain, dyspepsia and even deficiency of liposoluble vitamins in the long-term.
b) An amphetamine named *phentermine* (Adipex-P® from Teva Pharmaceuticals and Ionamin® from Medeva Pharmaceuticals) which can increase the blood pressure (also primary pulmonary hypertension and cardiovascular problems) and produce insomnia. Recently, a new antiobesity drug derived from the combination of *phentermine* and the antiepilectic *topiramate* (Qsymia® de Vivus Inc.) has been approved by the USFDA.

Thus, the need of finding effective and long-term safe pharmacological therapies for obesity continues being a primary objective for the international pharmaceutical industry and worldwide research.

### BRIEF DESCRIPTION OF THE INVENTION

The current invention refers to the design and development of oil-in-water nanoemulsions that can be used for the delivery of N-acylethanolamines, characterized by: a) an aqueous phase, b) an oily phase constituted by droplets of average diameter below 500 nanometers, c) an amphiphilic block copolymer and d) N-acylethanolamine.

Hence, a first aspect of the invention concerns a water-in-oil (w/o) nanoemulsion (from now on "nanoemulsion of the invention") that is appropriate for the administration of N-acylethanolamines. It comprises:
a. An aqueous phase;
b. An oily phase that is composed by oil droplets having a mean diameter below 500 nanometers;
c. An amphiphilic triblock copolymer; and
d. A compound with stoichiometry (I): 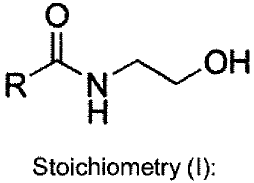
where the R-group is a linear alkyl (C11-C21) or linear alkenyl (C11-C21) with 1-6 double bonds and where the weight/weight (w/w) ratio copolymer/oil phase is in between 0.02 and 0.24. Preferably, this ratio (w/w) copolymer/oil phase is in between 0.02 and 0.16. More preferably, this ratio (w/w) copolymer/oil phase is in between 0.032 and 0.08. Even more preferably this ratio (w/w) copolymer/oil phase is 0.04.

In a preferable realization of the first aspect of the invention, the aqueous phase is at a concentration between 5% and 45% in weight by the total volume of the nanoemulsion (w/v).

In another preferable realization of the first aspect of the invention, the amphiphilic block copolymer is a poloxamer. In an even more preferable realization the copolymer is pluronics F68 and pluronics F127.

In another preferable realization of the first aspect of the invention, the oil within the oily phase is sunflower oil, peanut oil, cotton, castor oil, soya oil, safflower, palm oil, α tocopherol (vitamin E), isopropyl myristate, squalene or other combination. Preferably, the oil phase comprises sunflower oil. Even more preferable, the oil phase is sunflower oil.

In another preferred realization of the first aspect of the invention, the compound in stoichiometry (I) is N-oleoylethanolamine.

A second aspect of the invention refers to the procedure to obtain the nanoemulsion of the invention. It comprises: i) dissolving the copolymer/s in water under mild agitation; ii) the drop-by-drop addition of oil to water solution from i, under mechanical agitation, to generate a preemulsion; iii) to reduce the size of the droplets of the emulsion by repeatedly passing the coarse emulsion produced in ii, through a high pressure homogeneizer forming the nanoemulsion; iv) to add under mild agitation the compound (Stoichiometry (I)) to the nanoemulsion obtained in iii.

A third aspect of the invention concerns the application of the nanoemulsion of the invention for therapy usage.

A fourth aspect of the invention concerns the use of the nanoemulsion of the invention for the fabrication of a drug for the treatment and prevention of obesity. Alternatively, this fourth aspect refers to a nanoemulsion of the invention for its use in the treatment and prevention of obesity. Preferably, the nanoemulsion of the invention to be used in the treatment and prevention of obesity will be orally administered.

A fifth aspect of the invention concerns the use of the nanoemulsion of the invention for the preparation of a drug to be used in treatment and prevention of dyslipidemia. Alternatively, this fifth aspect of the invention concerns the nanoemulsion of the invention for its use in the treatment and prevention of dyslipidemia. Preferably, the nanoemulsion of the invention to be used in the treatment and prevention of obesity will be orally administered.

In a preferable realization of the fifth aspect of the invention, dyslipidemia will be selected from a list consisting in hypercholesterolemia, hypertriglyceridemia, hyperlipoproteinemia type I, IIa, IIb, III, IV or V, hyperquilimichronemia or combined hyperlipidemia.

A sixth aspect of the invention concerns a pharmaceutical composition which comprehends in the nanoemulsion of the invention and optionally one or more pharmaceutically acceptable excipients.

### BRIEF DESCRIPTION OF THE FIGURES

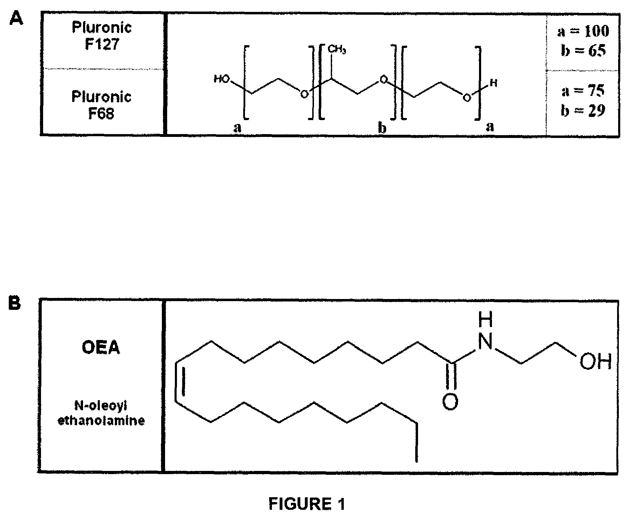

**Figure 1****.** Chemical structure of Pluronic F68 and Pluronic F127 **(A),** and OEA **(B).**

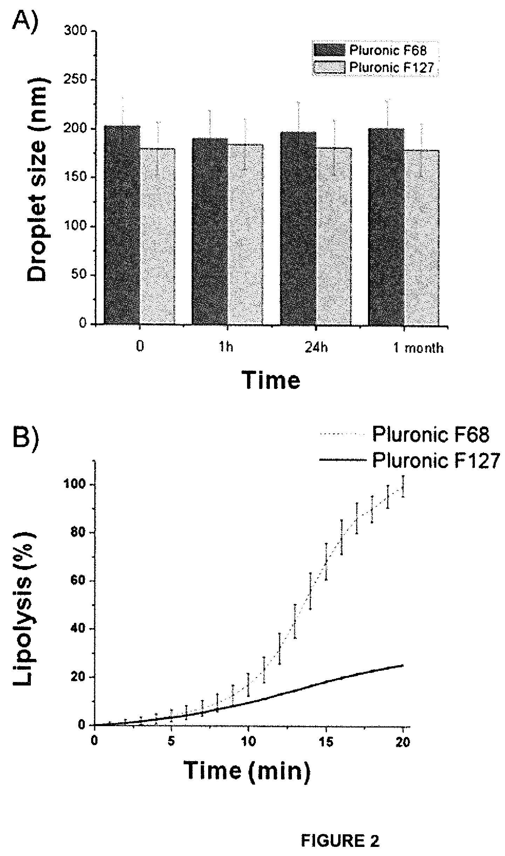

**Figure 2****.** Main properties of the nanoemulsions. **(A)** Mean droplet size and stability of the nanoemulsions of the invention were determined from Dynamic Light Scattering. **(B)** *In vitro* biodegradation of the nanoemulsions under duodenal conditions. This example was performed using the nanoemulsions described in the example #1 and example #2.

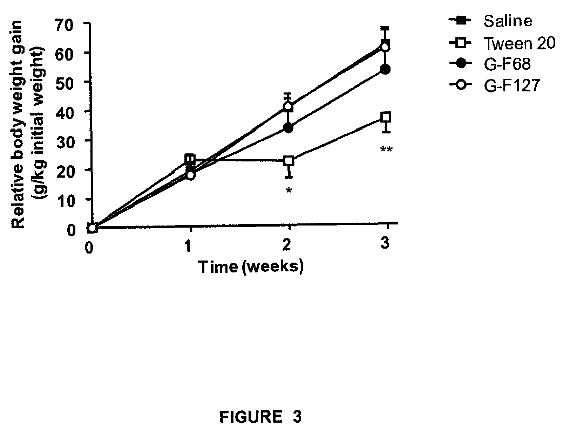

**Figure 3****.** Relative body weight gain in male Wistar rats (n=8 per group) after 3 weeks of daily administration (i.p.) of saline, Tween 20 (10% diluted in saline), nanoemulsions G-F68 (10% diluted in saline) or nanoemulsion G-F127 (10% diluted in saline). Points (■, □, ● y ○) are means ± SEM (represented by ┬ o ┴). Data were analyzed by two-way ANOVA (time and treatment) and a Bonferroni post-test. **p*<0.05 and ***p*<0.01 denote significant differences compared to control group (saline).

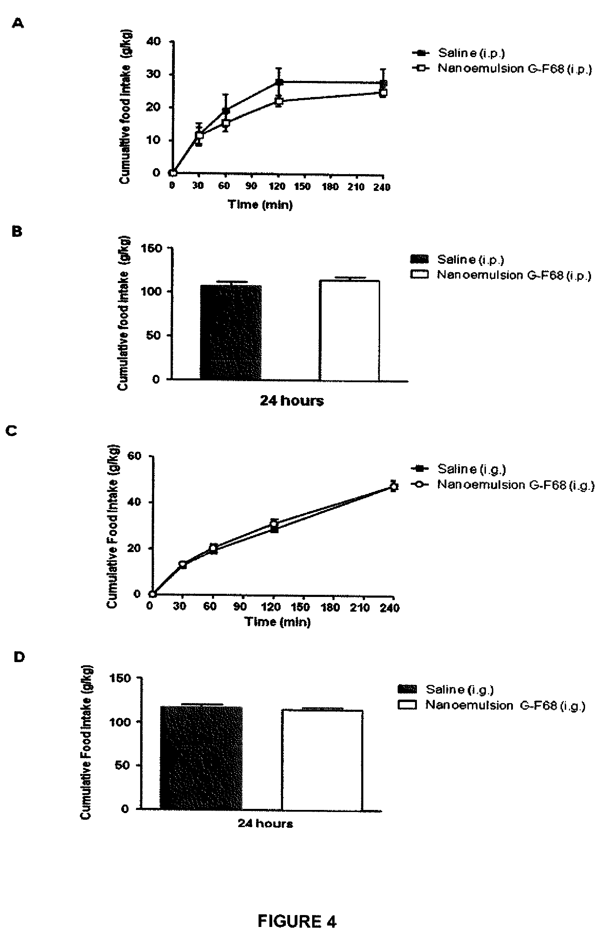

**Figure 4****.** Acute effect of intraperitoneal **(A, B)** or intragastric **(C,** D) administration of saline or nanoemulsion at 30 min, 60 min, 120 min, 240 min and 24 h on cumulative food intake in 24 h food-deprived Wistar rats (n=8 per group). Bars are means ± SEM. Points (■, □) are means ± SEM (represented by ┬ o ┴). Data were analyzed by two-way ANOVA (time and treatment) and a Bonferroni post-test.

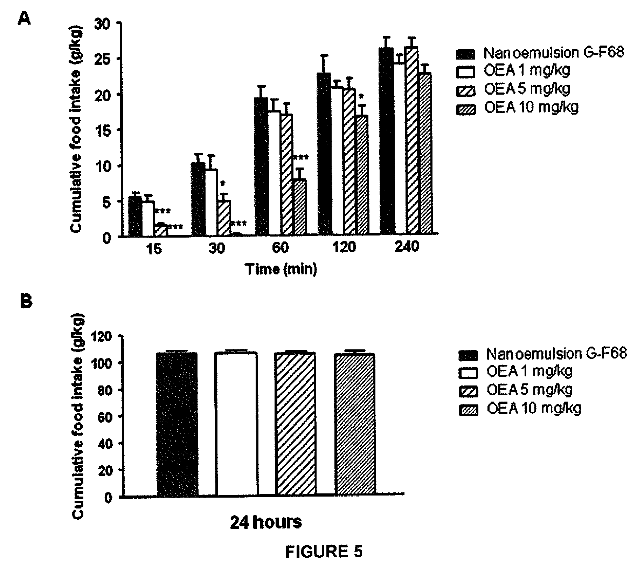

**Figure 5****.** Cumulative food intake in 24 h food-deprived Wistar rats (n=8 per group) after acute administration of OEA (i.p.) within nanoemulsion. **(A)** 15, 30, 60, 120 and 240 min. **(B)** 24 h. Bars are means ± SEM. Data were analyzed by two-way ANOVA (time and treatment) and a Bonferroni post-test. **p*<0.05 and ****p*<0.001 denote significant differences compared to control group (nanoemulsion).

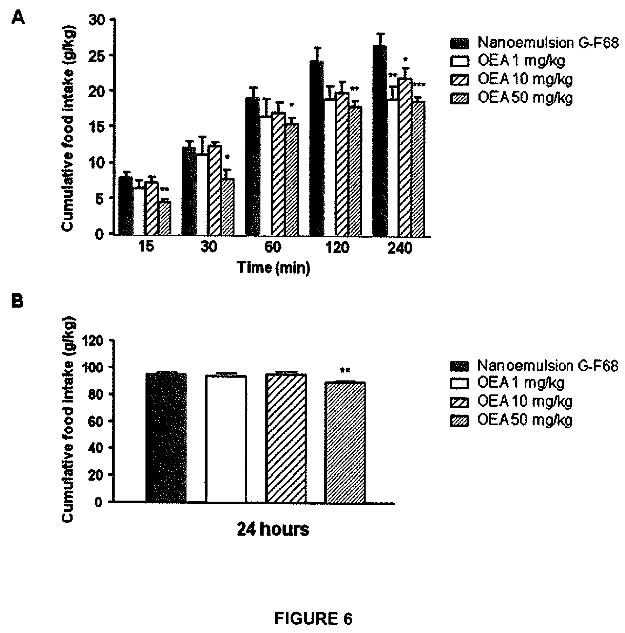

**Figure 6****.** Cumulative food intake in 24 h food-deprived Wistar rats (n=8 per group) after acute administration of OEA (via oral) within nanoemulsion. **(A)** 15, 30, 60, 120 and 240 min. **(B)** 24 h. Bars are means ± SEM. Data were analyzed by two-way ANOVA (time and treatment) and a Bonferroni post-test. **p*<0.05, ***p*<0.01 and ****p*<0.001 denote significant differences compared to control group (nanoemulsion).

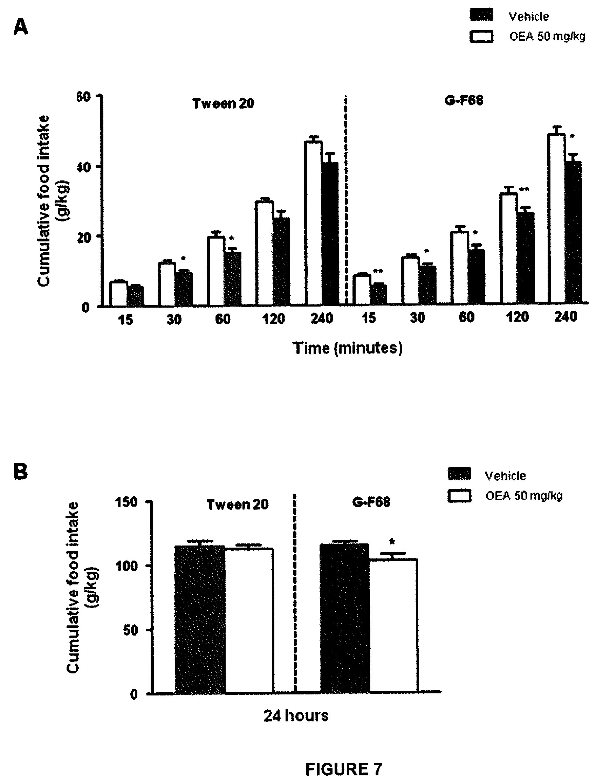

**Figure 7****.** Cumulative food intake in 24 h food-deprived Wistar rats (n=8 per group) after acute administration of OEA (50 mg/kg, via oral) diluted in Tween 20 or within nanoemulsion. **(A)** 15, 30, 60, 120 and 240 min. **(B)** 24 h. Bars are means ± SEM. Data were analyzed by two-way ANOVA (time and treatment) and a Bonferroni post-test. **p*<0.05 and ***p*<0.01 denote significant differences compared to their respective control group.

### DESCRIPTION OF THE INVENTION

The present invention faces the problem of finding long-term effective and safe pharmacological treatments against obesity. The present invention resolves this problem through the design and development of oil-in-water (o/w) nanoemulsions that are used as delivery system for N-acylethanolamines, and are characterized by (a) an aqueous phase, (b) an oil phase with a droplet size in the nanometer scale with a diameter under 500 nm, (c) an amphiphilic copolymer, and (d) the N-acylethanolamine.

It is known that the signaling system of N-acylethanolamines (NAEs) is a pharmacological target for the treatment of obesity. This system is involved in energy balance, appetite control and adiposity. This signaling system includes the ligands (NAEs, e.g. N-arachidonoylethanolamine, N-palmitoylethanolamine, N-oleoylethanolamine...) as well as the proteins and molecules involved in their synthesis, activity (cannabinoid receptors CB1 and CB2, the transient receptor potential vanilloid type-1 TRPV1, and the transcription factor PPAR alpha) and degradation. Concretely, the NAE N-oleoylethanolamine (OEA) has been described as a potent satiety factor at peripheral level (mainly in the adipose tissue and gastrointestinal tract), and it has been developed several synthetic derivatives of OEA that are able to induce satiety, modulate the body fat and prevent the excessive body weight gain. Although these compounds display a negligible toxicity, their low water solubility is a major limiting factor for their administration. At present, the *in vivo* administration of lipophilic compounds such as cannabinoids, vanilloids or NAEs has been performed using different substances as delivery system (vehicles). It has been reported effective results in experimental assays under chronic/subchronic administrations to animal models; however, there are still problems related to the development of drugs, including:
a) Organic solvents such as propylene glycol, dimethyl sulfoxide (DMSO), ethanol or methanol (usually dissolved in saline 30-95%). The main problem is their toxic level to use them as medication.
b) The surfactants Tween 20 and Tween 80 [Polyoxyethylene glycol (20/80) sorbitan monolaurate]. Although these compounds improve the problem related to the toxicity of organic solvents, it has been described liver damage and alteration of several parameters that are crucial in the evaluation of anti-obesity drugs (irritation, alteration of the immune system, reduction of body weight gain and food intake, hemolysis, altered circulating adiposity levels, increase of hepatic lipid content...).
c) Organic polymers such as carboxymethylcellulose have been used to be administered orally. However, drugs only display anti-obesity effect using high doses that might result in toxic problems and drug overdose.
d) Commercial emulsions, e.g. Tocrisolve® 100 (Tocris Bioscience) a formulation composed of soya oil, water and Pluronic F68, display a non-toxic profile as described in the datasheet. While these emulsions are suitable for systemic administration via intraperitoneal, intramuscular or subcutaneous, the oral administration is complicated due to the opposite conditions of the drugs within these emulsions related to the absorption in the gastrointestinal tract (pH, bile, presence of food, motility, enzymes such as the lipases...), affecting both the absorption and bioavailability of the drug.

However, the authors of the present invention have found that specific nanoemulsions characterized by (a) an aqueous phase, (b) an oil phase with a droplet size in the nanometer scale with a diameter under 500 nm, and (c) an amphiphilic copolymer, are useful as drug-delivery system for NAEs since these nanoemulsions do not display any of the problems previously mentioned. Thus, the authors of the present invention have evaluated *in vivo* this type of nanoemulsions. For this purpose, male Wistar rats have been treated with the nanoemulsions or other vehicles commonly used to solubilize hydrophobic drugs (e.g. Tween 20 (10% in saline v/v)) for 3 weeks. Using the saline group as control, the animals treated with the nanoemulsions of the present invention displayed an improvement of the toxicological profile compared to the animals treated with Tween 20 and it was similar to the profile displayed by the control group (see example 4).

It is important to remark that the nanoemulsion of the inventions have been synthesized from the previous identification of the stability/instability mechanisms of oil-in-water nanoemulsions. The structural information obtained by rheological tools has been correlated with the bulk macroscopic behavior, hence relating structure and functionality. In parallel to these works, a comparative study has been carried out on the hydrolisis reactions of the oils by the lipases, through the simulation of the degradation processes in the intestinal track using a pendant drop tensiometer. In this sense, for the development and selection of these nanoemulsion it has been studied the effect of the presence of bile salts, major components in the lipolysis process. It has been studied how gastric, duodenal and intravenous conditions affect the nanoemulsions, and specially the action of the corresponding lipases (lipolysis) because this action is directly related to the delivery location and rate of the drug in the organism as well as its subsequent adsorption. In this sense, the experimental work is shown in figure 2 using a range of nanoemulsions according to the specifications reported in examples 1 and 2, in other words, a nanoemulsion was used where the surfactant/oil ratio (w/w) is between 0.032 and 0.08. This way, the ratio between surfactant and oil was sufficient to completely cover the droplets with a size below 500 nm (i.e. a surfactant concentration larger than 0.5 % in weight/volume), but not too large to avoid destabilization of the nanoemulsion by depletion-flocculation (i.e. below 6 % in weight/volume), resulting in the optimal conditions regarding size, droplet coating and stability. Duodenal lipolysis simulation tests were carried out by mixing the previously described nanoemulsions with a duodenal digestion buffer that contains the main ingredients involved in a digestion process (bile salts, pancreatic lipase, sodium and calcium chlorides, etc) at similar concentrations to those existing in the organism. The technique employed consists in the measurement of the reduction of the retrodispersed light intensity (that is related to the size of the nanoemulsion droplets). As a result, when the lipolysis occurs, the triglycerides that compose the nanoemulsion droplets are converted in byproducts that exit the droplets are solubilized in the aqueous phase (monoglycerides and fatty acids). This results in a droplet size reduction that is directly related to a diminishing in the light intensity reduction retrodispersed by the nanoemulsion.

This change in the intensity ranges from a negligible value (0% lipolysis) to a maximum value (100% lipolysis). As observed in figure 2, the nanoemulsion of the invention has a typical size in the range of nanoemulsions and this size remains stable in time (see figure 2A). The nanoemulsion is also conveniently biodegraded when mixed with duodenal juices (see figure 2B) hence resulting in the delivery of the administered compounds in the required place. This way, along a series of experimentations, has determined that the ratio copolymer/oily phase in the nanoemulsion of the invention plays a key role in the achievement of long-term, stable nanoemulsions, from the point of view of the toxicological profile a good adsorption at duodenal level. As a result, the current invention is the first to assert that nanoemulsions comprising: a) and aqueous phase (b) an oil phase comprising oil droplets below 500 nm diameter and c) an amphiphilic block copolymer where the ratio (w/w) copolymer/oil phase is between 0.02 and 0.16, more preferably between 0.032 and 0.08, and even more preferably with the ratio approximately 0.04, are specially useful for the administration of N-acylethanolamine.

Hence, it has been demonstrated that the nanoemulsions of the invention are stable over time and they exhibit a low toxicity level and a good adsorption at intestinal level which makes these nanoemulsions an excelent pharmacological vehicle for the administration of N-acylethanolamine.

Hence, a first aspect of the invention concerns a water-in-oil (w/o) nanoemulsion (from now on " nanoemulsion of the invention ") that is appropriate for the administration of N-acylethanolamines. It comprises:
a. An aqueous phase;
b. An oily phase that is composed by oil droplets having a mean diameter below 500 nanometers;
c. An amphiphilic triblock copolymer; and
d. A compound with stoichiometry (I): 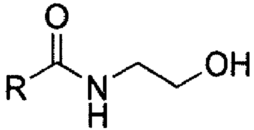 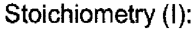
where the R-group is a linear alkyl (C11-C21) or linear alkenyl (C11-C21) with 1-6 double bonds and where the weight/weight (w/w) ratio copolymer/oil phase is in between 0.02 and 0.24. Preferably, this ratio (w/w) copolymer/oil phase is in between 0.02 and 0.16. More preferably, this ratio (w/w) copolymer/oil phase is in between 0.032 and 0.08. Even more preferably this ratio (w/w) copolymer/oil phase is approximately 0.04.

In the context of the present invention, (w/w) refers to the weight percentage of surfactant/oil phase.

Also, by nanoemulsions it is understood structures composed by oils of mixtures of oils that are acceptable in food applications, dispersed in water as nanometer sized droplets, that in their turn are stabilized by means of a copolymer that prevents droplet coalescence and flocculation. This kind of nanoemulsions is determined by their droplet mean size and distribution. According to the average droplet diameter, nanoemulsions are understood as those emulsions that exhibit a typical size in the size range of 20-500 nm, being micro- or macro-emulsions those having an average in the diameter range of 0.5-100 µm. Preferably, attending to the mean diameter of the droplet, nanoemulsions are understood by those emulsions having a mean diameter below 300 nm.

It is worth to remark that nanoemulsions in the current invention, exhibit a good physical stability after long resting times (without flocculation nor coalescence). Furthermore they do not require a vast amount of surfactant contrary to microemulsions. For example, for an oil-in-water emulsion (25%), a surfactant concentration in between 0.5% and 6% (w/v), better than 0.8% and 2%, would be sufficient to stabilize the nanoemulsion. This is a much smaller value if compared to typical values associated to microemulsions (10-20%), with the accompanying toxicity problems.

On the other hand, the particular percentages in the aqueous phase of the nanoemulsion will depend on the preparation method employed. Preferably the water phase will be at a concentration between 5% and 75% in weight per total volume (w/v). However, in any case, for the nanoemulsion of the invention to be stable over time, with low toxicity profile and good adsorption at intestinal level, it is necessary to keep the ratio (w/w) copolymer/oil phase between 0.02 and 0.16, more preferably this ratio is between 0.032 and 0.08, and even more preferably this ratio is 0.04.

On the other hand, it is worth to note that the surfactant employed in the preparation of the nanoemulsion of the inventions are amphiphilic block copolymers where by amphiphilic it is understood those molecules, substances, active principles, structures or their parts that exhibit both hydrophobic and hydrophilic characteristics.

According to IUPAC 1, block copolymers are substances formed by macromolecules constituted by blocks placed in a linear sequence. For this organism, a block is a portion of a macromolecule, that comprises several primary units, with at least one characteristic that is not present in adjacent units. There are copolymers formed by two or three blocks, that are typically represented as AB and ABA or BAB, where A and B are the polymeric blocks. Other kind of block copolymers are also possible, for instance; block copolymers constituted by the repetition of primary units formed by two blocks, (AB)n and by the incorporation to a two block polymer of a third chain (ABC). Other types block copolymers are graft and star copolymers.

Among the block copolymers with pharmaceutical applications we have to highlight amphiphilic copolymers. They consist in macromolecules formed by lipophilic blocks joined to hydrophilic blocks. This kind of block copolymers, with tensoactive properties, self assemble in micelles and crystal liquids that can act as excellent vehicles for active principles. Among the amphiphilic block copolymers most frequently used in pharmaceutical formulations one can find copolymers formed by blocks derived from ethylene oxide and propylene oxide. They are commonly known as poloxamers and their commercial names: Pluronic (Basf) and Synperonic (ICI). These copolymers have molecular masses in the range 1000-15000 and a block concentration of poly(ethylene oxide) between 10 and 80 wt%.

The hydration of the hydrophilic block of poly(ethylene oxide) is due to the easy arrangement with the water structure. The hydration is highly dependent on the temperature and therefore, the solubility and aggregation formation of block copolymers including this polar group can the regulated by temperature variations. These copolymers which are compatible and low-cost can adopt different arrangements when dispersed in water. These are dependent on the proportion of each of the blocks and the relative molecular mass. This proportion also determines the solubility and hydrophilic/lypophilic balance.

Pluronic copolymers are designated using letters and numbers. Letter "L" is used for the liquids, "P" for pastes and "F" (flake) for solids. The first digit, or the first two digits in numbers with three digits, multiplied by 300 is approximately equal to the mean relative molecular mass of the lypophilic part. The last digit, multiplied by 10 indicates the mass percentage of the hydrophilic part. Hence, for example, Pluronic F68 is a solid copolymer in which the mean relative molecular mass of the hydrophobic part is 1800 (6 x 300) and the mass percentage of the hydrophilic part is approximately 80% (8 x 10).

In another preferred point of the first aspect of this invention, the amphiphilic block copolymer of the nanoemulsion of the invention is a poloxamer, more preferably, the amphiphilic block copolymer is the pluronic block copolymer F68 or the pluronic block copolymer F127.

On the other side, as previously stated, the nanoemulsion of the invention is characterized by an oily phase that comprises oil droplets with a mean diameter below 500 nm, preferably below 300 nm. In this sense it is important to remark that in the context of this invention, the mean diameter is determined by means of the Dynamic Light Scattering (DLS) technique that gives the hydrodynamic diameter of a equivalent sphere that diffuses light similarly to the particles that are being measured. This technique provides the mean diameter of the droplets and the polydispersity index. Furthermore, this is the method recommended by the United States Pharmacopeia for the measurement of the size of the droplets in injectable lipidic emulsions (method 729 USP).

Additionally, oils used to formulate the nanoemulsion of the invention can be selected among one or more natural, semisynthetic and synthetic oils of pharmaceutical usage such as oils of animal origin, vegetable origin, hydrocarbons and silicone oils. Appropriate oils for the current invention include, but are not limited to, mineral oil, squalene oil, flavour oils, silicone oils, essential oils, water insoluble vitamins, isopropyl stearate, butyl stearate, octyl palmitate, Cetyl palmitate, tridecil behenate, azodicarboxylate adipate, dioctyl sebacate, mentil anthranilate, Cetyl octanoate, Octyl salicylate, isopropyl myristate, cetoles of dicarpato of neopentyl glycol, Cerafilos ®, decile oleate, C12-C15alquil lactates, Cetyl lactate, sodium lauryl lactate, isostearil neopentanoato, myristylammonium lactate, isocetil Stearoyl stearate, octildodecil Stearoyl stearate, oils hydrocarbons, isoparaffin, fluid paraffin, isododecane, vaseline, argan oil, rapeseed oil, chile oil, coconut oil , corn oil, oil from cotton, flax oil, seed oil, oil of mustard, olive oil, palm oil, fractionated palm oil, oil peanut, castor oil, seed oil pine oil poppy seed, seed oil pumpkin, bran oil of rice, safflower, tea oil, truffle oil, vegetable oil, apricot kernel oil, jojoba oil, macadamia oil, wheat germ oil, oil of almond, soybean oil, sesame oil, oil of hazelnut, sunflower oil, oil of hemp, bois oil, Kukui nut, oil of avocado, walnut, fish oil, Berry oil, oil of pepper of Jamaica, oil of Juniper, seed oil, almond seed oil, oil of anise seed, celery seed oil , oil of cumin seed, seed oil of nutmeg, oil of Basil leaf, oil of bay leaf, oil of cinnamon, oil of common Sage leaf blade, leaf of eucalyptus leaf oil of lemon, leaf oil of melaleuca, oil of oregano, leaf patchouli, peppermint leaf oil, oil pine needle oil , leaf oil of Rosemary, oil of Spearmint, tea tree leaf oil, leaf of thyme, oil of tea leaf of Canada, oil of Roman Sage, clove oil, geranium flower oil, camomile, flower, oil of hyssop flower, oil of Jasmine Flower, oil of lavender flower, oil of flower of mauka, flower of Marjoram, orange blossom oil, oil of flower of pink, oil of flower of ylang-ylang, bark oil, cassia bark, oil of cinnamon bark, bark oil of Sassafras, wood oil, wood oil of camphor, oil of cedar, oil of rosewood, sandalwood oil, ginger oil, resin oil, castor oil, myrrh oil , oil of skin, skin of Bergamo, oil from skin of grapefruit, lemon zest, oil of lime zest, orange peel, Tangerine skin, root oil, oil of valerian oil, oleic acid, linoleic acid, oleil alcohol, alcohol isostearilo, ethyl, Miglyol ®, Labrafil ®, Labrafac ®, oleate Rylo ®, Peceol ® and Maisine ®, derived synthetic or semi-synthetic and combinations thereof. I.e., in the context of the present invention, the oil in the oil phase of the nanoemulsions can consist in a single oil or a mixture of different oils, preferably selected from the above list. Preferably the oil consists in sunflower oil. Even more preferably, oil is sunflower oil, i.e. the oil in the oily phase is sunflower oil.

On the other hand, as it has been previously defined, the nanoemulsions of this invention comprise an N-acylethanolamine as active principle. The term "active principle" refers to some other substance that is used to the treatment, cure, prevention or diagnosis of diseases or that is used to improve both the physical and mental welfare in humans and animals. Thus, the nanoemulsions of the present invention are suitable for their use in the treatment or prevention of both obesity and dyslipidemia.
The N-acylethanolamines (NAEs) that are used in the present invention are fatty acid amides that act as endogenous lipid mediators. Thus, in the context of the present invention, a NAE is a compound with the following chemical structure: 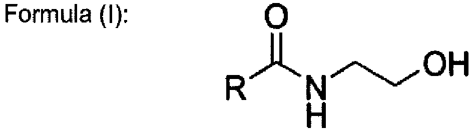 wherein R is a linear alkyl (C11-C21) or linear alkenyl (C11-C21) with 1-6 double bonds. In a preferred aspect of the present invention, R can be selected from anyone of the following groups:
1. CH₃(CH₂)₁₀,
2. CH₃(CH₂)₁₂,
3. CH₃(CH₂)₁₄,
4. CH₃(CH₂)₁₅,
5. CH₃(CH₂)₁₆,
6. CH₃(CH₂)₁₈,
7. CH₃(CH₂)₃CH=CH(CH₂)₇.
8. CH₃(CH₂)₅CH=CH(CH₂)₇,
9. CH₃(CH₂)₈CH=CH(CH₂)₄.
10. CH₃(CH₂)₇CH=CH(CH₂)₇.
11. CH₃(CH₂)₅CH=CH(CH₂)₉,
12. CH₃(CH₂)₄CH=CHCH₂CH=CH(CH₂)₇,
13. CH₃CH₂CH=CHCH₂CH=CHCH₂CH=CH(CH₂)₇,
14. CH₃(CH₂)₄CH=CHCH_{z}CH=CHCH₂CH=CHCH₂CH=CH(CH₂)₃,
15. CH₃CH₂CH=CHCH₂CH=CHCH₂CH=CHCH₂CH=CHCH₂CH=CH(CH₂)₃,
16. CH₃(CH₂)₇CH=CH(CH₂)₁₁,
17. CH₃CH₂CH=CHCH₂CH=CHCH₂CH=CHCH₂CH=CHCH₂CH=CHCH₂CH=CH(OH₂)₂

A specific NAE is the N-oleoylethanolamine (OEA); therefore in another preferred aspect of the first aspect of the invention, the NAE is OEA. The term OEA, just like it is used in the present invention, refers to a NAE which structure is described in the Figure 1 B and that is produced naturally to regulate food intake and body weight gain in all vertebrates. OEA is an endogenous ligand of the peroxisome proliferator-activated receptor alpha (PPARα) that is involved in the stimulation of the lipolysis.

On the other hand, a second aspect of this invention refers to a procedure to elaborate the nanoemulsion of the invention. In this regard, it is important to point out that the procedures to obtain the nanoemulsions of the invention are easy methods to avoid drastic conditions such as high temperatures. Moreover, it is not necessary to perform any type of chemical reaction since the obtaining of the system involved non-covalent interactions. Thus, the integrity of the molecules incorporate to the system that are susceptible of degradation is preserved. In this regard, for the synthesis of the nanoemulsions of the invention is necessary to proceed to: i) dissolve the copolymer(s) in water under light stirring; ii) add the oil, drop by drop, to the aqueous phase produced in the previous stage; iii) reduce the drop size of the emulsion, the pre-emulsion will pass through a high pressure homogenizer several times; iv) add, under slight stirring, a compound of the formula (I) to the nanoemulsion of the stage (iii).

In addition, another aspect of the present invention refers to the use of the nanoemulsion of the invention for therapy. Concretely, it is referred as a pharmaceutical composition that includes the nanoemulsion of the invention, and optionally, one or more suitable pharmaceutical excipients. Especially, the addition of N-acylethanolamines to the nanoemulsions of the invention produce compositions with specific characteristics related to their composition, properties and morphology. Thus, these compositions are excellent candidates in the therapeutic area, specifically, in the treatment or prevention of both obesity and dyslipidemia. The type of N-acylethanolamine to add to the nanoemulsions of the invention will be the N-acylethanolamine with suitable pharmacotherapeutic properties according to the therapeutic use to which the formulation will be use. Thus, in a specific execution, the N-acylethanolamine is the N-oleoylethanolamine.

Therefore, another aspect of the invention refers to a composition that includes the nanoemulsion of the invention for its use in the treatment or prevention of obesity. Alternatively, the invention also refers to the use of a composition that includes the nanoemulsion of the invention for the production of a medication for the treatment or prevention of obesity. Preferably, the nanoemulsion of the invention will be administered orally to its use in the treatment or prevention of obesity.

In another aspect, the invention refers to a composition that includes the nanoemulsion of the invention for its use in the treatment or prevention of dyslipidemia. Alternatively, the invention also refers to the use of a composition that includes the nanoemulsion of the invention for the production of a medication for the treatment or prevention of dyslipidemia. Preferably, the nanoemulsion of the invention will be administered orally to its use in the treatment or prevention of dyslipidemia.

Additionally, in another aspect, the invention is related with a method for the treatment or prevention of a metabolic disease in a subject that consists of the administration to this subject of a composition which is the nanoemulsion of the invention.

The term "obesity", according to how is used in the present invention, refers to the obesity definition provided by the WHO and it is based in the body mass index (BMI) which consists of the relationship between the weight of a person in kilograms divided by the square of the height in meters. According this criteria, a BMI under 18.5 kg/m² is considered underweight, a BMI of 18.5 to 24.9 kg/m² is considered normal weight, a BMI of 25 to 29.9 kg/m² is considered overweight class I, a BMI of 30 to 39.9 kg/m² is considered obesity or overweight class II, and a BMI over 40 kg/m² is considered morbid obesity. Alternatively, there are other methods to define the obesity in individuals such as the waist circumference (in cm) measured at midpoint between the lowest rib and the iliac crest, skin-folds thickness and the bioimpedance, which is based on the lean tissue is a better conductor of electricity than fat.

The term "dyslipidemia", according to how is used in the present invention, refers to some pathological condition that is characterized by an alteration on the lipid metabolism with the consequent alterations on the concentration of lipids (cholesterol, triglycerides and other similar) and lipoproteins (high density lipoproteins) in the blood. Among the dyslipidemias that can be treated with the methods of the present invention are, with no limitation, hypercholesterolemias, hypertriglyceridemias, hyperlipoproteinemias types I, IIa, IIb, III, IV and V, hyperchylomicronemias, combined hyperlipidemias...

The term "subject", according to how is used in the present invention, includes living organisms, including human beings (male and female of any age or race); animals (e.g. monkeys, cows, sheeps, horses, pigs, goats, dogs, cats, mice, rats and transgenic animals). In a preferred aspect, the subject is a human being.

The quantity of active agents (NAE) that will be effective in the treatment of obesity or dyslipidemia can be determined by standard clinical methods based on the present description. Moreover, *in vitro* assays should be optionally used to help to identify the optimum dosages. The precise dose to be used in the formulation will also depend on the administration route and the gravity of the illness, and it will be decided according to the opinion of the doctor and the circumstances of each subject.

For the oral administration, a therapeutically effective dose can be estimated using *in vitro* assays. For example, it is possible to formulate a dose in animal models to get a circulating concentration range that includes the IC50 (it is a measure of the effectiveness of a drug that shows the substance concentration causing the 50% inhibition of a specific biological function) determined by a cell culture. This information can be used to determine the effective doses in humans in a precise manner. The initial dosage can be also estimated from *in vivo* data, for instance, animal models using techniques that are well-known. Somebody with a normal experience in the technique will be able to easily optimize the administration to humans based on the animal data.

Next, in order to provide a better understanding of the characteristics and benefits of the present invention, several examples will be mentioned in an explanatory manner to complete the previous description, but it does not mean that the present invention is limited to them.

### THE FOLLOWING EXAMPLES ARE GIVEN TO ILLUSTRATE THE INVENTION AND SHOULD NOT BE CONSTRUED TO LIMIT THE SCOPE OF THE INVENTION

### EXAMPLE 1. Preparation and obtaining of nanoemulsion G-F68

Sunflower oil was purchased from Sigma-Aldrich Co. (St. Louis, MO, USA) and purified through activated magnesium silicate to remove free fatty acids. Poloxamer Pluronic F68 is a polymeric surfactant with a molecular weight of 8400 Da acquired from Sigma-Aldrich Co. (St. Louis, MO, USA). 0.3 g poloxamer Pluronic F68 were added to 22.2 g of purified MilliQ water (0.054 mS) and dissolved in it with the help of a magnetic stirrer. Then, 7.5 g of sunflower oil were added drop by drop while shaken mechanically through a high-speed Heidolph Diax 900 homogeneizer, keeping the agitation for 4 minutes at 13000 rpm, forming the pre-emulsion. This pre-emulsion was immediately homogenized by a highpressure Emulsiflex-C3 (Avestin, CA, USA) homogenizer to 103 MPa. This last process was repeated 8 times.

### EXAMPLE 2. Preparation and obtaining of nanoemulsion G-F127

The Pluronic F127 (poloxamer P407) surfactant is a triblock copolymer with a molecular weight of 12600 g/mol, acquired from Sigma-Aldrich. The method of preparation of the nanoemulsions containing this surfactant is similar to the explained in example 1 for the Pluronic F68, with the only modification of the quantity of Pluronic employed (in grams). In order to ensure a proper coating of the nanoemulsion droplets, the amount of Pluronic F127 will be equivalent in moles to the Pluronic F68. As a result, because of the larger molecular weight, the amount to be added in grams will be higher. Thus, in this case the amount of Pluronic F127 to be added was 0.45 g.

### EXAMPLE 3. Preparation of the formulation with OEA

Starting from the G-F68 nanoemulsion formulation, the appropriate amount of OEA is added according to the dose to use. In a vial containing a volume of nanoemulsion corresponding to a 5% (v/v) of the total volume of the solution, a previously weighted amount of OEA (solid state) is added. This vial will be kept in agitation at a constant temperature in the range 35-45 °C for 30 minutes. After this time, the nanoemulsion containing OEA will be subjected to sonication in a conventional water bath for 5 minutes controlling the temperature. Next, the vial will be agitated again under dropwise addition of saline (5% NaCl) to complete the remaining 95% of total volume of the working solution. This preparation will take place in fresh the same day in vivo experimental work.

**EXAMPLE 4. Biological activity of nanoformulations with OEA and G-F68.** Next, the invention will be illustrated by *in vivo* assays performed by the authors of the present invention, which show the efficacy of the formulations of this invention.

### Experimental animals

The experiments were performed on male Wistar rats weighing 300-350 g. The animals were housed individually under a 12 h light/dark cycle in a room with temperature and humidity control. The rats had *ad libitum* access to food and water, except during the feeding studies. Prior to the experiments, animals were acclimated to the handling.

### Drugs and doses

For the long-term feeding study, it was prepared a series of solutions with potential use to liposoluble drugs related to feeding behavior:
a) Normal saline
b) Tween 20 (10% in saline v/v) was obtained from Tocris Bioscience (Bristol, UK)
c) Nanoemulsion G-F68 (10% in saline v/v)
d) Nanoemulsion G-F127 (10% in saline v/v)

All the solutions were injected intraperitoneally (i.p.) at a dose volume of 1ml/kg every day for 3 weeks.

For the acute feeding studies, the nanoemulsion G-F68 or Tween 20 were prepared in 5% in saline.

Regarding the acute studies with OEA, the drug was obtained from Tocris Bioscience (Bristol, UK). It was administered in a solution contained the nanoemulsion G-F68 (5%) and saline (95%) prepared in fresh the same day of the experiment. The doses tested were:
a) 1, 5 and 10 mg/kg of OEA for the intraperitoneal acute studies. The volume of the administration was 1 ml/kg.
b) 1, 10 and 50 mg/kg of OEA for the oral acute studies. The volume of the administration was 3 ml/kg.

### EXAMPLE 5. Long-term feeding study and determination of fat liver content and metabolites in plasma of the new nanoemulsions: toxicity and metabolic effects

The nanoemulsions G-F68 and G-F127 have been evaluated in vivo in male Wistar rats for 3 weeks compared to another vehicle commonly used to liposoluble drugs: Tween 20 (10% in saline v/v). Using the saline group as control, the animals treated with the nanoemulsions of the present invention displayed an improvement of the toxicological profile compared to the animals treated with Tween 20 and it was similar to the profile displayed by the control group.

### 1. Body weight changes

The administration of Tween 20 induced a significant weight gain reduction as compared with the saline-treated group that started to be significant on the second week of exposure (**p*<0.05) and this difference was increased on the third week (***p*<0.01). By contrast, the administration of the nanoemulsions had no effect on body weight gain in comparison with control rats treated with saline (Figure 3).

### 2. Biochemical determinations in plasma

Animals were anesthetized (pentobarbital 200 mg/kg, i.p.) and sacrificed by decapitation after the treatment with the different solutions. Blood was collected and centrifuged (2,000xg, 15 min, 25°C) to obtain the plasma. Samples were stored at -20°C. The biochemical analysis showed that Tween 20 treatment significantly increased the level of cholesterol (***p*<0.01), triglycerides (**p*<0.05) and transaminases [GPT (***p*<0.01) and GOT (***p*<0.01)] compared with the saline group. However, the animals treated with the new nanoemulsions displayed an improvement on these biochemical parameters compared with the Tween 20 group: a) Animals receiving the nanoemulsions G-F68 displayed higher level of cholesterol than saline-treated rats (**p*<0.05) rats, but the treatment with this nanoemulsion induced a significant decrease on triglyceride (###*p*<0.001) and transaminase levels [GPT (#*p*<0.05) and GGt (#*p*<0.05)] compared with Tween 20-treated animals, b) The nanoemulsion G-F127 reduced the level of creatinine (#*p*<0.05), cholesterol (##*p*<0.01) and GPT (#*p*<0.05). See Table 1.

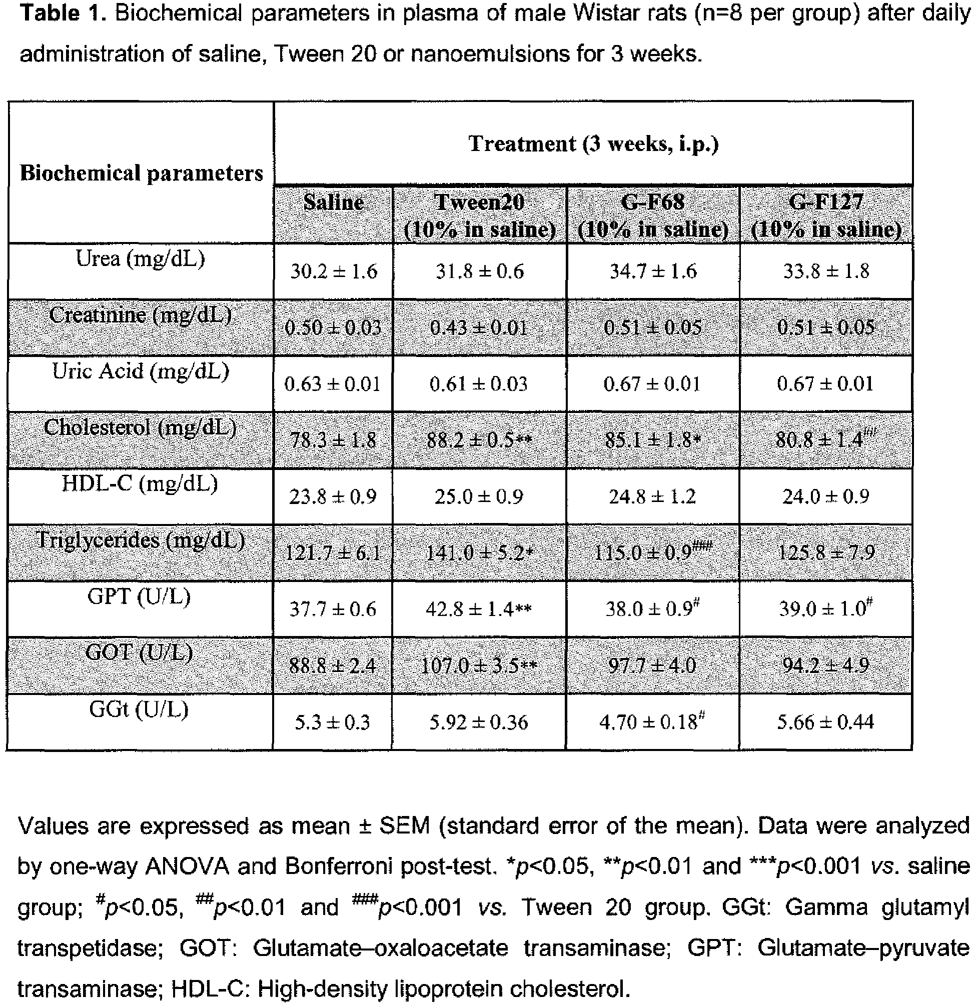

### 3. Liver fat content

Total lipids were extracted from frozen liver samples (from animals anesthetized and sacrificed by decapitation after the treatment with the different solutions) using a mixture of chloroform-methanol (2:1 [v/v]). Tween 20 treatment produced a significant increase in fat content (**p*<0.05) compared with the saline group. Animals receiving the new nanoemulsion G-F68 intraperitoneally displayed similar level of hepatic lipid content than saline-treated rats, but significant lower than Tween 20-treated animals (#*p*<0.05). See Table 2

**Table 2. Hepatic lipid content of male Wistar rats (n=8 per group) after daily administration of saline, Tween 20 or nanoemulsion G-F68 for 3 weeks.**

| | **Treatment (3 weeks, i.p.)** | | |
|---|---|---|---|
| | **Saline** | **Tween 20 (10% in saline)** | **G-F68 (10% in saline)** |
| **Total fat content (% of tissue weight)** | 3.999 ± 0.091 | 4.850 ± 0.295* | 3.857 ± 0.186^{#} |

Values are expressed as mean ± SEM (standard error of the mean). Data were analyzed by one-way ANOVA and Bonferroni post-test. **p*<0.05 *vs.* saline group; ^{#}*p*<0.05 *vs.* Tween 20 group.

### EXAMPLE 6. Acute effects of OEA on feeding behavior after systemic or oral administration of OEA

The hypophagic actions of the N-acylethanolamine OEA were evaluated using the nanoemulsion G-F68 as delivery system (vehicle). For these experiments, we used animals habituated to the experimental protocols. To this end, 48 h before each experiment, the bedding was removed and replaced by small food containers into the cage for 4 h. After this initial phase, the animals were deprived of food for 24 h, but had free access to water. The different treatments were administered 15 min prior access to food. Animals were then returned to their home cages and a measured amount of food (30-40 g) was placed into the cages (t = 0 h). Food pellets were weighed at time intervals of 15, 30, 60, 120 and 240 min, and 24 h after the acute treatment.

### 1. Effects of nanoemulsion G-F68 on feeding behavior

First, the acute intraperitoneal administration (1 ml/kg, i.p.) as well as the acute oral administration (3 ml/kg, i.g.) of G-F68 had no effect on food intake in food-deprived rats compared with the saline group (Figure 4). After 24 h of the treatment, the cumulative food intake was evaluated again and there was no difference on cumulative food intake between saline- and nanoemulsion-treated rats.

### 2. Effects of the intraperitoneal administration of OEA dissolved in the nanoemulsion G-F68

As the previous results indicated that this nanoemulsion lack of *in vivo* effects on feeding, OEA were administered via intraperitoneal using this new nanoemulsion as a delivery system (5% in saline). Food intake was evaluated at different time intervals for 24 h after the treatment. Thus, the use of these nanoemulsions reproduced the anorexic activity previously described for this lipid mediator (Rodríguez de Fonseca y col., 2001; Fu y col., 2003).

As shown in Figure 5, the systemic administration of OEA produced a significant reduction on food intake at the doses of 5 mg/kg (****p*<0.001 at 15 min; **p*<0.05 at 30 min) and 10 mg/kg (****p*<0.001 at 15, 30 and 60 min) compared with the nanoemulsion-treated group. 24 h after the OEA and nanoemulsion administration, the different doses of OEA had no effects on cumulative food intake in comparison with the vehicle group (G-F68).

### 3. 2. Effects of the oral administration of OEA dissolved in the nanoemulsion G-F68

On the other hand, food-deprived rats were treated with OEA administered orally (i.g.) in a volume of 3 ml/kg. These animals were previously habituated to the intragastric administration using saline.

Food intake was dose-dependently reduced by oral administration of different doses of OEA compared with the control group (rats treated with the nanoemulsions G-F68). OEA-treated rats displayed a significant decrease in food intake with all doses tested: 1 mg/kg (***p*<0.01 at 240 min), 10 mg/kg (**p*<0.05 at 240 min) and 50 mg/kg of OEA (***p*<0.01 at 15 min, **p*<0.05 at 30 and 60 min; ***p*<0.01 at 120 min, ****p*<0.001 at 240 min, and ***p*<0.01 at 24 h ) compared with the group treated with the nanoemulsions G-F68 (Figure 6).

### 4. Acute effect of OEA prepared in Tween 20 or nanoemulsion G-F68 on feeding behavior after oral administration

For this experiment, food-deprived rats received an acute treatment (via oral, i.g.) of OEA prepared in Tween 20 or nanoemulsion G-F68. OEA was administered in a volume of 3 ml/kg at the most effective dose previously tested (50 mg/kg).

As shown in Figure 7, while OEA-treated rats displayed a significant decrease in food intake only at 30 and 60 min (**p*<0.05) compared with the vehicle Tween 20 group, OEA within the nanoemulsion G-F68 induced a prolonged and significant reduction of feeding at all times (***p*<0.01 at 15 min, **p*<0.05 at 30 and 60 min; ***p*< 0.01 at 120 min, **p*<0.05 at 240 min and 24 h).

It should be pointed out that the present results show that the oral administration of OEA using the nanoemulsion G-F68 as a delivery system inhibits food intake at effective doses lower than the doses previously described in the literature, as well as the effect is longer than previous studies. These data demonstrate that these nanoformulations might release the OEA more gradually because the composition of the nanoemulsion.

Therefore, the administration of these new nanoemulsions containing NAEs or other lipophilic drugs related with these molecules represents an effective and safe drug-delivery system. This has a direct use in the biomedical and pharmaceutical fields because these NAEs might be administered in a proper way to therapeutic purposes. For instance, the satiety effect of OEA using this nanoformulation might be used to reduce the food intake and, therefore, to decrease the related health problems: obesity, diabetes, cardiovascular diseases, etc.

## Claims

1. An oil/water nanoemulsion suitable for the administration of N-acylethanolamines, that includes:
a. An aqueous phase
b. An oil phase that contains an oil formed by drops with a diameter lower than 500 nanometers.
c. An amphiphilic block copolymer; and
d. A compound of formula (I) 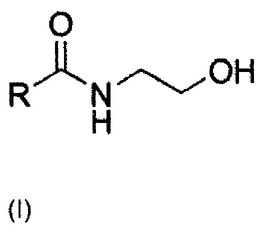
Where the R group is a lineal alkyl (C11-C21) or a lineal alkylene (C11-C21) with 1-6 double bonds and with a copolymer/oil phase ration (p/p) between 0.02 and 0.16.

2. The nanoemulsion according to the claim 1, where a copolymer/oil phase ration (p/p) is between 0.032 and 0.08.

3. The nanoemulsion according to the claim 1, where the copolymer concentration is a concentration of 1% in weigh of the total volume of the nanoemulsion (w/v).

4. The nanoemulsion according to the previous claims, where the amphiphilic block copolymer is a poloxamer.

5. The nanoemulsion according to the previous claims, where the oil of the oil phase is chosen from a list that consists of sunflower, peanut, cotton, olive, castor-oil plant, soybean, safflower, palm, α tocopherol (vitamin E), isopropyl miristate, esqualene oil or any combination of them.

6. The nanoemulsion according to the previous claims, where the compound of formula (I) is N-oleylethanolamine.

7. Procedure to the attainment of the nanoemulsion just is defined in the claim 1, that includes: i) to dissolve the copolymers in water under soft shaking; ii) to add the oil drop by drop to the aqueous phase produced in the step i), under mechanic shaking, producing a pre-emulsion; iii) to reduce the size of the emulsion drops making pass in repeated times the pre-emulsion of the step ii) through an homogenizing of high pressure, forming thus the nanoemulsion; iv) to add under soft shaking to the nanoemulsion of the step iii) a compound of the formula (I).

8. The nanoemulsion according to any of claims 1 to 6 for its use in therapy.

9. Use of the nanoemulsion according to any of claims 1, to 6, to manufacture a medicament for the treatment or prevention of obesity.

10. Use of the nanoemulsion according to any of claims 1 to 6, to manufacture a medicament for the treatment or prevention of dyslipidemia..

11. Use of the nanoemulsion according to any of claims 1 to 6, to manufacture a medicament for the treatment or prevention of dyslipidemia, where aforesaid dyslipidemia is selected from the list that consists of hypercholesterolemia, hypertriglyceridemia, hyperlipoproteinemias types I, IIa, IIb, III, IV and V, hyperchylomicronemias or combined hyperlipidemias.

12. Pharmaceutical composition which comprises the nanoemulsion of any of claims 1 to 6, and optionally, one or more pharmaceutically acceptable excipients.

13. The nanoemulsion according to any of claims 1 to 6, to its use in the treatment or prevention of obesity by oral administration.

14. The nanoemulsion according to any of claims 1 to 6, to its use in the treatment or prevention of dyslipidemia by oral administration.

15. The nanoemulsion according to any of claims 1 to 6, to its use by oral administration in the treatment or prevention of dyslipidemia, where aforesaid dyslipidemia is selected from the list that consists of hypercholesterolemia, hypertriglyceridemia, hyperlipoproteinemias types I, IIa, lib, III, IV and V, hyperchylomicronemias or combined hyperlipidemias.
